(19) Europäisches Patentamt
European Patent Office
Office européen des brevets

(11) Numéro de publication: **0 410 901 A1**

(12) **DEMANDE DE BREVET EUROPEEN**

(21) Numéro de dépôt: **90420282.7**

(51) Int. Cl.5: **C07C 27/16**, B01J 23/46

(22) Date de dépôt: **13.06.90**

(30) Priorité: **29.06.89 FR 8909003**

(43) Date de publication de la demande: **30.01.91 Bulletin 91/05**

(84) Etats contractants désignés: **CH DE FR GB IT LI NL**

(71) Demandeur: **RHONE-POULENC CHIMIE**
**25, quai Paul Doumer**
**F-92408 Courbevoie Cédex(FR)**

(72) Inventeur: **Lecomte, Jean-Pierre**
**24, rue Boileau**
**F-69006 Lyon(FR)**
Inventeur: **Costantini, Michel**
**10, rue du Docteur Bonhomme**
**F-69003 Lyon(FR)**
Inventeur: **Laucher, Dominique**
**205, Avenue Jean Jaurès**
**F-69007 Lyon(FR)**

(74) Mandataire: **Varnière-Grange, Monique et al**
**RHONE-POULENC CHIMIE Service Brevets**
**Chimie Centre de Recherches des Carrières**
**B.P. 62**
**F-69192 Saint-Fons Cédex(FR)**

(54) **Procédé d'oxydation catalytique d'alcanes en mélanges d'alcools et de cétones.**

(57) La présente invention concerne l'oxydation en phase liquide d'alcanes par le peroxyde d'hydrogène, en un mélange d'alcools et de cétones, avec conservation du nombre d'atomes de carbone, en présence d'osmium ou d'un composé de l'osmium.

EP 0 410 901 A1

Xerox Copy Centre

## PROCEDE D'OXYDATION CATALYTIQUE D'ALCANES EN MELANGES D'ALCOOLS ET DE CETONES

La présente invention a pour objet un procédé d'oxydation catalytique d'alcanes, en mélanges d'alcools et de cétones, avec conservation du nombre d'atomes de carbone.

L'oxydation catalytique d'alcanes par le peroxyde d'hydrogène est une réaction connue en elle-même et divers systèmes catalytiques ont déjà été proposés pour condure cette réaction.

Ainsi, dans le brevet américain n° 3,660,503 il est proposé un procédé de production d'alcools à prédominance primaires par traitement d'un hydrocarbure, choisi parmi les paraffines linéaires ou ramifiées, les cycloparaffines renfermant au moins un substituant éthyle, avec une quantité stoéchimétrique de peroxyde d'hydrogène comme seul agent hydroxylant en présence d'un catalyseur à base d'un métal du groupe VIII de la classification périodique sur un support, la température de réacteur étant comprise entre 150 et 350° C et la pression, entre la pression atmosphérique et environ 50 atm. La réaction est conduite en pratique en phase vapeur et, si un mélange d'alcools à prédominance primaires est produit, on note toutefois que la réaction en cause se déroule avec une ou des coupures du substrat et conduit à la formation d'alcools dont le nombre d'atomes de carbone est inférieur à celui renfermé par le substrat.

Ces coupures s'analysant au plan économique comme des pertes en carbone compromettent l'utilisation à l'échelle industrielle du procédé en cause.

Il était donc nécessaire de proposer un procédé d'oxydation catalytique d'alcanes par le peroxyde d'hydrogène qui permette de conserver le squelette carboné de la molécule à oxyder.

La présente invention a donc pour objet un procédé d'oxydation d'alcanes en un mélange d'alcools et de cétones avec conservation du nombre d'atomes de carbone, par le peroxyde d'hydrogène caractérisé en ce qu'on opère à une température inférieure à 150° C, en phase liquide et en présence d'une quantité efficace d'osmium ou d'un composé de l'osmium.

Par alcanes, substrats dans le cadre du présent procédé on entend des hydrocarbures saturés répondant à la formule (I) ci-après :

RH (I)

dans laquelle R représente :

- un groupement alkyle linéaire ou ramifié qui comporte de 1 à 30 atomes de carbone,
- un groupement cycloalkyle qui comporte de 3 à 12 atomes de carbone dans le cycle, éventuellement substitué par un ou plusieurs groupements alkyles qui renferment au maximum 4 atomes de carbone,
- un groupement polycycloalkyle comportant de 2 à 5 cycles dont chaque cycle peut renfermer de 3 à 12 atomes de carbone, ou
- un groupement alkyl- ou cyclyalkylaromatique qui comporte de 7 à 30 atomes de carbone.

Plus spécifiquement R représente :

- un groupement alkyle linéaire ou ramifié qui comporte de 1 à 12 atomes de carbone,
- un groupement cycloalkyle qui comporte de 5 à 12 atomes de carbone dans le cycle,
- un reste d'alkylbenzène dans lequel le groupe alkyle renferme au maximum 4 atomes de carbone, ou
- un reste de cycloalkylbenzène dans lequel le groupe cycloalkyle renferme de 5 à 8 atomes de carbone.

A titre d'exemples d'alcanes oxydables dans le cadre du présent procédé, on peut citer : le méthane, l'éthane, le propane, l'isobutane, l'isopentane, le butane, l'hexane, l'octane, le cyclopentane, le cumène, le toluène, la tétraline, la décaline, le cyclododécane et le pinane.

Dans le cadre du présent procédé on recourt également à du peroxyde d'hydrogène.

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvant organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl-tertiobutyléther.

Comme indiqué en tête du présent mémoire, on obtient dans le cadre du présent procédé un mélange renfermant au moins l'alcool et au moins la cétone correspondant à l'alcane utilisé comme matière première ou à la partie alkylique du substrat aralkylique. Ainsi à partir du cyclohexane on obtiendra un mélange de cyclohexanol et de cyclohexanone qui sont les intermédiaires utiles de diverses fabrications (acide adipique, caprolactame) mélange communément désigné par OLONE. De même au départ d'éthylbenzène on obtiendra un mélange de phényl-1 éthanol et d'acétophénone utile pour la fabrication du styrène.

La mise en oeuvre du présent procédé requiert la présence d'osmium ou d'un composé de l'osmium.

N'importe quelle source d'osmium peut être utilisée dans le cadre de la présente invention. On peut, en effet, engager l'osmium sous forme métallique, le cas échéant, finement divisée ou déposée sur un support tel le charbon actif. Conviennent également à la mise en oeuvre dudit procédé, des composés de l'osmium dans lesquels l'osmium est au degré nul d'oxydation tel le triosmiumdodécacarbonyle. On peut également utiliser des composés minéraux de l'osmium dans lesquels l'osmium présente l'un quelconque des degrés d'oxydation 2 à 8. A titre d'exemples de tels composés on peut citer : OsO, $Os_2O_3$, $OsO_2$, $OsO_4$, $OsCl_3$, $K_2OsO_4$, $NaOsF_6$, $OsOCl_4$, $K_2OsO_4(OH)_2$, $OsCl_4$ et $OsOF_5$.

Il est également possible d'utiliser des composés organiques ou des complexes de l'osmium tels le tétracyclohexylosmium, le tétra(cyclohexyloxy) osmium ou le complexe Os(TPP)(CO)(Pyridine) et notamment, des complexes porteurs de coordinats denses en atome d'azote (par exemple tri- ou tétraazotés) tels les coordinats qui présentent le squelette de la tétraphénylporphyrine. La plupart des complexes en cause libèrent in situ dans les conditions de la réaction une forme minérale de l'osmium soit par dégradation des coordinats soit par décomplexation.

De préférence, on utilise l'une quelconque des formes d'osmium du groupe ci-après :
Os/C, $Os_3(CO)_{12}$, OsO, $Os_2O_3$, $OsO_2$, $OsO_4$ et $OsCl_3$.

Le tétroxyde d'osmium convient particulièrement bien à la mise en oeuvre de l'invention.

La quantité d'osmium à mettre en oeuvre n'est pas critique et elle peut varier dans de larges limites. Pour une bonne mise en oeuvre de l'invention elle sera d'au moins $10^{-6}$ mole d'osmium par mole de peroxyde d'hydrogène et on n'observe aucun avantage à dépasser une quantité de $10^{-1}$ mole d'osmium par mole de peroxyde d'hydrogène. Cette quantité est, de préférence, comprise entre $10^{-2}$ et $10^{-5}$ mole d'osmium par mole de peroxyde d'hydrogène.

Le rapport molaire du peroxyde d'hydrogène à l'alcane peut lui aussi varier dans de larges limites ; un minimum de 0,001 % molaire est toutefois préconisé pour pouvoir observer un taux de transformation appréciable de l'alcane, ce rapport pouvant atteindre 100 %. De préférence, ce rapport est compris entre 0,5 et 25 %.

Bien entendu la réaction peut être conduite avec un large excès d'alcane qui sert alors aussi de diluant. On peut également opérer en présence de diluants ou solvants non oxydables dans les conditions de la réaction, tels le benzène et le chlorobenzène.

Selon une variante avantageuse du présent procédé, la réaction est conduite en présence d'un diluant choisi parmi les alcools saturés, les diols et polyols saturés, l'eau et leurs mélanges.

Les alcools saturés qui conviennent à la mise en oeuvre du présent procédé répondent à la formule générale (II) ci-après :

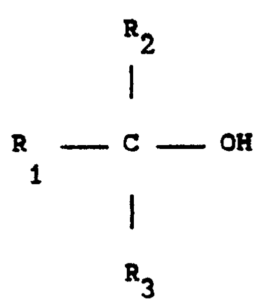

(II)

dans laquelle :
- $R_1$ à $R_3$, identiques ou différents, représentent :
* des atomes d'hydrogène,
* des groupements alkyles linéaires ou ramifiés qui comportent de 1 à 30 atomes de carbone,
* des groupements cycloalkyles qui comportent de 3 à 12 atomes de carbone,
* des groupements alkyl- ou cuycloalkylaromatiques qui comportent de 7 à 30 atomes de carbone, ou
* des groupements aryles, éventuellement substitués par un ou deux groupements alkyles qui renferment au maximum 4 atomes de carbone, groupements aryles qui comportent de 6 à 20 atomes de carbone ;
- deux des groupements $R_1$ à $R_3$ peuvent en outre former ensemble un radical unique divalent alkylène renfermant de 4 à 10 atomes de carbone,
- les trois groupements $R_1$ à $R_3$ peuvent en outre former ensemble un radical unique trivalent polycyclique renfermant de 6 à 20 atomes de carbone.

Les diols et le polyols saturés comportent un squelette répondant à la formule générale (II) ci-avant sur lequel est introduit au moins un groupe hydroxyle supplémentaire et, de préférence, au maximum 6

groupes hydroxyles supplémentaires.

A titre d'exemples de tels diluants on peut citer : le méthanol, l'éthanol, l'isopropanol, le tertiobutanol, l'hexanol-1, l'octanol-1, le dodécanol-1, le cyclohexanol, le diméthylphénylcarbinol, l'éthylèneglycol, le propanediol-1,3 et le diméthyl-2,4 dihydroxy-2,4 pentane.

Bien entendu l'alcool saturé utilisé comme diluant peut être de même nature ou de nature différente de l'alcool produit par la réaction ; il est également possible d'utiliser comme diluant un mélange renfermant un alcool endogène et un alcool exogène au milieu réactionnel.

De préférence, on utilise l'eau ou un alcool saturé répondant à la formule (II) ci-avant dans laquelle $R_1$ à $R_3$, identiques ou différents représentent des groupements alkyles linéaires qui comportent de 1 à 4 atomes de carbone ou, un atome d'hydrogène.

Le tertiobutanol convient plus particulièrement bien à la mise en oeuvre du procédé.

Comme indiqué ci-avant il est possible d'utiliser des mélanges de diluants et en particulier un mélange d'alcool saturé (ou de diol ou polyol saturé) et d'eau.

La quantité de diluant ou de mélange de tels diluants peut varier dans de larges limites : une influence sensible est constatée dès lors que cette quantité représente de l'ordre de 2 % en poids de l'alcane à oxyder et aucun effet positif n'est observé lorsque cette quantité dépasse 200 % en poids de l'alcane. De bons résultats sont obtenus pour une quantité de diluant (s) comprise entre 2 et 100 % en poids de l'alcane.

Lorsqu'on utilise un mélange d'alcool et d'eau, la quantité d'eau n'est pas critique ; elle peut varier dans de larges limites.

Selon la teneur précise en eau du milieu réactionnel, la nature précise de l'alcool et/ou de l'alcane on peut observer la présence d'une seule phase ou de deux phases : une phase organique et une phase aqueuse. La présence d'un tel système biphasique notamment en fin de réaction est un autre avantage lié au procédé de la présente invention dans la mesure où l'on peut séparer les produits d'oxydation et l'alcane qui n'a pas réagi du système catalytique dont la majeure partie se trouve en fin de réaction dans la phase aqueuse, par décantation ou extraction. La phase aqueuse résiduelle peut être aisément utilisée, le cas échéant après traitement, pour catalyser une nouvelle réaction d'oxydation.

La Demanderesse a constaté qu'il est également avantageux d'opérer en présence d'un mélange tampon de telle sorte que le pH de la phase aqueuse soit maintenu entre 2 et 14. Pour ce faire, elle préconise notamment l'addition au milieu réactionnel d'un ou plusieurs composés choisis parmi : les hydroxydes des métaux alcalins, les oxyacides minéraux ou organiques et leurs sels des métaux alcalins ou des métaux alcalino terreux et en particulier, l'acide acétique et ses sels, l'acide phosphorique et ses sels, et, l'acide borique et ses sels.

La température de réaction dépend de la nature précise de l'alcane à oxyder. Elle est généralement comprise entre 50 et 150 °C et, de préférence entre 70 et 150 °c.

On opère à pression atmosphérique ou le cas échéant sous pression supérieure à la pression atmosphérique de manière à maintenir les constituants du mélange réactionnel en phase liquide.

La durée de réaction (ou le temps de séjour) généralement comprise entre quelques minutes et plusieurs heures peut être ajustée, compte-tenu des objectifs de production, de la quantité de catalyseur et des autres paramètres réactionnels.

En fin de réaction les produits peuvent être récupérés par tout moyen approprié, par exemple par distillation.

Les exemples ci-après illustrent l'invention.

Les conventions suivantes y sont utilisées :

- TT désigne : le taux de transformation de du peroxyde d'hydrogène engagé à la réaction.
- RT désigne : le rendement en un produit par rapport au peroxyde d'hydrogène transformé.
- T : désigne la température

EXEMPLES 1 à 4 :

Dans un tube de verre, muni d'un bouchon à opercule siliconé et d'un barreau magnétique, on introduit :

- 3,9 g d'une solution préparée

contenant :

- 3,0 g (36 mmol) de cyclohexane
- 0,8 g (11 mmol) de tertio-butanol
- 0,040 g de solution aqueuse de peroxyde d'hydrogène à 70 % en poids (0,8 mmol)

- 180 µg (0,7 µmol) de $OsO_4$
- éventuellement un cocatalyseur comme indiqué dans le tableau (I) ci-après :

Le tube bouché est placé dans un bain d'huile thermostaté. Le milieu réactionnel est agité magnétiquement, et sa température mesurée par une sonde thermométrique.

L'avancement de la réaction est suivi par dosage iodométrique d'échantillons.

En fin de réaction, l'excès éventuel de peroxyde d'hydrogène est réduit par une solution de triphénylphosphine dans le dichlorométhane, et la solution obtenue analysée par chromatographie en phase gazeuse (étalon interne : orthodichlorobenzène).

Les conditions particulières ainsi que les résultats obtenus figurent dans le tableau (I) ci-après dans lequel,
- RT(ONE) désigne le rendement en cyclohexanone par rapport à $H_2O_2$ consommée.
- RT(OL) désigne le rendement en cyclohexanol par rapport à $H_2O_2$ consommée.

TABLEAU I

| Ex N° | cocatalyseur | | | $H_2O_2$ 70% | | $OsO_4$ µmol | T °C | Durée h | TT (%) | RT ONE (%) | RT OL (%) |
|---|---|---|---|---|---|---|---|---|---|---|---|
| | nature | mg | µmol | mg | mmol | | | | | | |
| 1 | - | - | - | 42 | 0,88 | 0,76 | 92 | 30 | 76 | 4 | 45 |
| 2 | acides<br>hexanoïque<br>adipique | <br>1,6<br>1,0 | <br>14<br>7 | 41 | 0,84 | 0,75 | 92 | 95 | 80 | 3 | 32 |
| 3 | TEA | 2,0 | 20 | 40 | 0,80 | 0,77 | 87 | 26 | 100 | 6 | 48 |
| 4 | pyridine | 1,6 | 21 | 37 | 0,76 | 0,75 | 87 | 49 | 82 | 2 | 28 |
| TEA : triéthylamine | | | | | | | | | | | |

EXEMPLE 5 :

Selon le mode opératoire décrit ci-avant, on charge :
- 3,4 g d'une solution préparée contenant :
. 2,7 g (31 mmol) de n-hexane
. 0,68 g (0,2 mmol) de tertiobutanol
. 0,035 g d'une solution aqueuse de peroxyde d'hydrogène à 70 % en poids (0,7 mmol)
. 680 µg (2,6 µmol) de $OsO_4$

Après 16 heures de chauffage à 92 °C, on mesure par iodométrie :
- TT = 48 %.

L'excès d'eau oxygénée est réduit par une solution de triphénylphosphine dans le dichlorométhane. De l'éthanol est ajouté pour homogénéiser le milieu qui est ensuite analysé par chromatographie en phase gazeuse (étalon interne : orthodichlorobenzène). Les rendements en divers produits (calculés par rapport à $H_2O_2$ consommée) sont les suivants :

| | |
|---|---|
| - Hexanone-1 et 2 = | 2 % |
| - Hexanone-3 = | 1 % |
| - Hexanol-1 = | 1,9 % |
| - Hexanol-2 = | 9,9 % |
| - Hexanol-3 = | 10,3 % |

EXEMPLES 6 à 10 :

Dans un tricol de verre, muni d'un réfrigérant, d'un compte-bulles, d'un thermomètre et d'un opercule en silicone, on introduit, après purge à l'argon :

- cumène : 29,7 g (248 mmol)
- le cosolvant, comme indiqué dans le tableau (II) ci-après
- 7,73 micromol d'osmium, sous la forme indiquée dans le tableau
- $H_2O_2$ en solution aqueuse à 70 % comme indiqué dans le tableau (II).

Le tricol est chauffé par bain d'huile thermostaté, et le milieu réactionnel agité magnétiquement.

L'avancement de la réaction est suivi par dosage iodométrique d'échantillons.

En fin de réaction, le milieu réactionnel est analysé par chromatographie en phase gazeuse (étalon interne : orthodichlorobenzène).

Les conditions particulières ainsi que les résultats obtenus figurent dans le tableau (II) ci-après dans lequel

. ACPH désigne l'acétophénone
. DMPC désigne le diméthylphénylcarbinol (phényl-2 propanol-2).

TABLEAU II

| Ex N° | composé d'osmium | cosolvant | | $H_2O_2$ 70% | | T °C | Durée h | TT (%) | RT ACPH (%) | RT (DMPC) (%) |
|---|---|---|---|---|---|---|---|---|---|---|
| | | nature | g | g | mmol | | | | | |
| 6 | $Os_3(CO)_{12}$ | t-Butanol | 8,2 | 0,59 | 12,2 | 87 | 29 | 100 | 7,6 | 27,5 |
| 7 | $OsO_4$ | i-Propanol | 8 | 0,59 | 12,2 | 84 | 18 | 100 | 1,1 | 7,1 |
| 8 | $OsO_4$ | Ethanol | 7,9 | 0,58 | 12,0 | 82<br>95 | 106 | 100 | 7,1 | 30 |
| 9 | $OsO_4$ | triéthylphosphate | 11,5 | 0,19 | 3,9 | 86 | 23 | 100 | n.d. | 64 |
| 10 | $OsO_4$ | tétraéthyl urée | 11,8 | 0,21 | 4,3 | 86 | 3,2 | 100 | 2,3 | 12,4 |
| n.d. = non déterminée | | | | | | | | | | |

EXEMPLE 11 :

Selon le mode opératoire décrit dans les exemples 6 à 10, dans le cas du cumène, on charge :

- 30,8 g (290 mmol) d'éthylbenzène
- 8,0 g (108 mmol) de tertio-butanol
- 0,65 g d'une solution à 40 mmol/l d'$OsO_4$, dans le cyclohexane (32 µmol)
- 0,38 g d'une solution aqueuse d'$H_2O_2$ à 70 % en poids (7,8 mmol)

Après six heures à 87° C, le milieu réactionnel est analysé. Les résultats obtenus sont les suivants :

| | |
|---|---|
| TT = | 100 % |
| RT (Acétophénone) = | 3 % |
| RT (Phényl-1 éthanol) = | 15 % |
| RT (Phényl-2 éthanol) = | 0,5 % |

EXEMPLE 12 à 16 :

Dans un réacteur en Pyrex de 200 ml, muni d'un réfrigérant, d'un barreau magnétique, d'un septum et

d'une arrivée d'argon, on charge :
- cyclooctane distillé : 32,1 g (300 mmol)
- alcool tertiaire : selon tableau (III)
- $OsO_4$ en solution dans le cyclooctane : selon tableau (III)

Le mélange est porté à température, avec agitation.

On coule, en trois minutes :
- $H_2O_2$ sous forme d'une solution aqueuse à 68 % en poids : 0,40 g (8,0 mmol)

Le taux de transformation de $H_2O_2$ est suivi par iodométrie d'échantillons.

En fin de réaction le milieu réactionnel est analysé par chromatographie en phase gazeuse (étalon : orthodichlorobenzène).

Les conditions particulières ainsi que les résultats obtenus figurent dans le tableau (III) ci-après dans lequel :

ONE désigne la cyclooctanone
OL désigne le cycloactanol
RM désigne le rapport molaire $OsO_4/H_2O_2$

TABLEAU III

| Ex n° | cosolvant | | $OsO_4$ µmol | RM | T °C | durée (h) | TT (%) | RT ONE | RT OL |
|---|---|---|---|---|---|---|---|---|---|
| | nature | g | | | | | | | |
| 12 | tBuOH | 7,6 | 800 | $1,0.10^{-1}$ | 90 | 2 | >95 | 6 | 52 |
| 13 | tBuOH | 7,6 | 90 | $1,1.10^{-2}$ | 87 | 2,3 | 100 | 3,8 | 77,2 |
| 14 | tBuOH | 7,6 | 10 | $1,2.10^{-3}$ | 88 | 6,3 | 100 | 4,6 | 77,3 |
| 15 | tAmOH | 8,5 | 9,9 | $1,2.10^{-3}$ | 99 | 1,0 | 100 | 6,7 | 55,3 |
| 16 | tAmOH | 13 | 0,94 | $1,1.10^{-4}$ | 99 | 5,5 | 98 | 8,7 | 56,7 |

tBuOH = tertiobutanol
tAmOH = méthyl-2 butanol-2 (alcool tertio amylique)

EXEMPLE 17 :

Dans un réacteur en Pyrex de 200ml, lavé avec $HNO_3$, muni d'un réfrigérant, d'un barreau magnétique, d'un septum et d'une arrivée d'argon, on charge :
- cyclooctane distillé : 21 g (188 mmol)
- alcool tertiaire selon tableau (IV)
- tampon 1 M dans l'eau selon tableau : 2,5 g (2,5 ml)

Le mélange est porté, sous argon, à la température indiquée dans le tableau, puis on coule successivement :
- $H_2O_2$ sous forme d'une solution aqueuse à 68 % en poids : 0,27 g (5,5 mmol)
- $OsO_4$ en solution dans le cyclooctane : 0,75 µmol

L'avancement de la réaction est suivi par iodoméotrie d'échantillons prélevés dans le milieu hétérogène bien agité. En fin de réaction, le milieu réactionnel est homogénéisé par ajout de tertio-butanol, et analysé par chromatographie en phase gazeuse (étalon interne : orthodichlorobenzène).

Les conditions particulières ainsi que les résultats obtenus figurent dans le tableau IV ci-après dans lequel :

ONE désigne le cyclooctanone
OL désigne le cycloactanol

TABLEAU IV

| Ex n° | alcool tertiaire | | Tampon 1 M | | T °C | durée (h) | TT (%) | RT ONE | RT OL |
|---|---|---|---|---|---|---|---|---|---|
| | nature | g | nature | pH à 20°C° | | | | | |
| 17 | - | - | $CH_3COOH/NaOH$ | 5,4 | 95 | 2,5 | 98,2 | 12,3 | 11 |
| 18 | tAmOH | 8,2 | $H_3PO_4/NaOH$ | 7,2 | 87 | 3,1 | 98,4 | 5,8 | 11 |
| 19 | tAmOH | 8,2 | $CH_3COOH/NaOH$ | 5,4 | 87 | 1,2 | 100 | 7,7 | 10,2 |

## Revendications

1° - Procédé d'oxydation catalytique d'alcanes en un mélange d'alcools et de cétones, avec conservation du nombre d'atomes de carbone, par le peroxyde d'hydrogène, caractérisé en ce qu'on opère en phase liquide, à une température inférieure à 150°C et en présence d'une quantité efficace d'osmium ou d'un composé de l'osmium.

2° - Procédé selon la revendication 1, caractérisé en ce que l'alcane a pour formule générale :

RH (I)

dans laquelle R représente :

- un groupement alkyle linéaire ou ramifié qui comporte de 1 à 30 atomes de carbone,
- un groupement cycloalkyle qui comporte de 3 à 12 atomes de carbone dans le cycle, éventuellement substitué par un ou plusieurs groupements alkyles qui renferment au maximum 4 atomes de carbone,
- un groupement polycycloalkyle comportant de 2 à 5 cycles dont chaque cycle peut renfermer de 3 à 12 atomes de carbone, ou
- un groupement alkyl- ou cyclyalkylaromatique qui comporte de 7 à 30 atomes de carbone.

3° - Procédé selon la revendication 1 ou 2, caractérisé en ce que l'alcane est choisi parmi les composés répondant à la formule (I) donnée dans la revendication 2, dans laquelle R représente :

- un groupement alkyle linéaire ou ramifié qui comporte de 1 à 12 atomes de carbone,
- un groupement cycloalkyle qui comporte de 5 à 12 atomes de carbone dans le cycle,
- un reste d'alkylbenzène dans lequel le groupe alkyle renferme au maximum 4 atomes de carbone, ou
- un reste de cycloalkylbenzène dans lequel el groupe cycloalkyle renferme de 5 à 8 atomes de carbone.

4° - Procédé selon l'une quelconque des revendications précédentes caractérisé en ce qu'on opère en présence d'un diluant choisi parmi les alcools saturés, les diols et polyols saturés, l'eau et leurs mélanges.

5° - Procédé selon la revendications 4, caractérisé en ce qu'on opère en présence d'un alcool de formule :

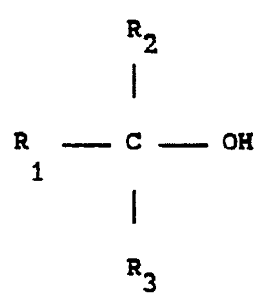

(II)

dans laquelle :

- $R_1$ à $R_3$, identiques ou différents, représentent :

* des atomes d'hydrogène,
* des groupements alkyles linéaires ou ramifiés qui comportent de 1 à 30 atomes de carbone,
* des groupements cycloalkyles qui comportent de 3 à 12 atomes de carbone,
* des groupements alkyl- ou cuycloalkylaromatiques qui comportent de 7 à 30 atomes de carbone, ou
* des groupements aryles, éventuellement substitués par un ou deux groupements alkyles qui renferment

au maximum 4 atomes de carbone, groupements aryles qui comportent de 6 à 20 atomes de carbone ;
- deux des groupements $R_1$ à $R_3$ peuvent en outre former ensemble un radical unique divalent alkylène renfermant de 4 à 10 atomes de carbone,
- les trois groupements $R_1$ à $R_3$ peuvent en outre former ensemble un radical unique trivalent polycyclique renfermant de 6 à 20 atomes de carbone.

6° - Procédé selon la revendication 5, caractérisé en ce que l'alcool répond à la formule (II) dans laquelle $R_1$ à $R_3$, identiques ou différents représentent des groupements alkyles linéaires qui comportent de 1 à 4 atomes de carbone ou, un atome d'hydrogène.

7° - Procédé selon l'une quelconque des revendications 4 à 6 caractérisé en ce que l'alcool représente de 2 à 200 % et, de préférence de 2 à 100 % en poids de l'alcane.

8° - Procédé selon l'une quelconque des revendications 4 à 7 caractérisé en ce qu'on opère en présence d'un mélange d'alcool et d'eau.

9° - Procédé selon l'une quelconque des revendications 4 à 8, caractérisé en ce que l'alcool utilisé est le tertiobutanol.

10° - Procédé selon l'une quelconque des revendications précédentes caractérisé en ce que la quantité d'osmium est comprise entre $10^{-2}$ et $10^{-5}$ mole par mole de peroxyde d'hydrogène.

11° - Procédé selon l'une quelconque des revendications précédentes caractérisé en ce que le peroxyde d'hydrogène représente de 0,001 à 100 % (molaire) de l'alcane, et de préférence, de 0,01 à 25 % (molaire).

12° - Procédé selon l'une quelconque des revendications précédentes caractérisé en ce que la température de réaction est comprise entre 70 et 150 °C.

13° - Procédé selon l'une quelconque des revendications précédentes caractérisé en ce que l'osmium est introduit sous forme d'osmium métallique, le cas échéant déposé sur un support, sous forme d'un composé minéral de l'osmium ou sous forme d'un complexe organique de l'osmium libérant dans le milieu réactionnel une forme minérale de l'osmium.

14° - Procédé selon l'une quelconque des revendications précédentes caractérisé en ce que l'osmium est introduit sous forme de son tétroxyde.

15° - Procédé selon l'une quelconque des revendications précédentes caractérisé en ce que l'alcane utilisée est le cyclohexane.

Office européen des brevets

# RAPPORT DE RECHERCHE EUROPEENNE

Numero de la demande

EP 90 42 0282

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-C-1 272 923 (CELANESE CORP.)<br>* revendication 1 * | 1 | C 07 C 27/16<br>B 01 J 23/46 |
| D,A | US-A-3 660 503 (BLOCH)<br>* colonne 4, paragraphe 1 * | 1 | |
| A | BE-A- 702 933 (CAPORTE CHEMICALS)<br>* revendications * | 1 | |
| A | AU-B- 406 801 (I.C.I.)<br>* revendication 1 * | 1 | |
| A | HOUBEN-WEYL: "METHODEN DER ORGANISCHEN CHEMIE"<br>4. édition, tome VI/Ia, page 178, 1981, Impr. Georg Thieme Verlag, Stuttgart, DE * page 178, paragraphe 5 * | 1 | |
| P,A | EP-A-0 331 590 (RHONE-POULENC)<br>* revendications * | 1 | |
| | | | **DOMAINES TECHNIQUES RECHERCHES (Int. Cl.5)** |
| | | | C 07 C 27/00<br>B 01 J 23/00<br>C 07 C 35/00<br>C 07 C 29/00 |

Le présent rapport a été établi pour toutes les revendications

| Lieu de la recherche | Date d'achèvement de la recherche | Examinateur |
|---|---|---|
| BERLIN | 02-10-1990 | PROBERT C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)